# EUROPEAN PATENT APPLICATION

(11) **EP 4 445 884 A1**
(43) Date of publication of application: **16.10.2024**
(21) Application number: 23167957.2
(22) Date of filing: 14.04.2023
(51) Int. Cl.: A61F 13/15

(54) **FLUSHABLE ABSORBENT PAD AND COMBINATION WITH REUSABLE CHASSIS**

(71) Applicant: W. Pelz GmbH & Co. KG, 23812 Wahlstedt/Holstein (DE)
(72) Inventor: WALLERT, Matthias, 23795 Klein Rönnau (DE); RÖTTGER, Henning, 24568 Kaltenkirchen (DE)
(74) Representative: Plischke, Manfred

(57) **Abstract**

The present invention is an absorbent pad suitable to be flushed, such as in a toilet and especially adapted to be fitted into a chassis, such that a hygiene article system is formed.

## Description

### Field of the invention

The present invention relates to an absorbent pad suitable to be flushed, such as in a toilet and especially adapted to be fitted into a chassis, such that a hygiene article system is formed.

### Background

Apart from washable cloth diapers, two-piece diaper systems comprising a reusable and washable outer layer and a disposable insert have long been used. Over the last decades, disposable diapers have significantly improved both ease of application and liquid handling performance, which further led to healthier skin of a wearer even at extended wear times, not only for babies but also for incontinent adults as well as for menstruating women. Especially for the latter two categories, absorbent pads are broadly used for lower liquid handling requirements, such as light to medium incontinence or lower menstrual flow conditions, whereby such pads are typically inserted into regular underwear, optionally attached thereto by releasable adhesive, or - mostly in hospitals or care facilities - elasticated pants. In particular for mobile people, discreetness during wear times and ease of replacement become highly relevant. However, with an increased environmental awareness, disposable products have come under scrutiny for using non-renewable resources and/or for an increase carbon footprint in the overall lifecycle, including treatment of the used products. Also, the disposability of hygiene articles, such as wipes, has been broadly discussed and for flushability stringent requirements have been developed.

US3489149 (Larson) describes a re-usable and washable panty for menstrual wear having in the crotch portion a pocket for an optional absorbent insert or pad, wherein the user-oriented portion of the pocket is formed from a first hydrophobic, yet moisture-passing layer for improved skin dryness oriented towards the wearer and a hydrophilic layer oriented towards the insert, if present, or the panty material, which has some tendency to retain moisture for the case when not used in combination with an insert pad.. The insert may be disposable.

In EP2376046, a wearable absorbent article to be worn by a wearer about the lower torso is described, comprising a re-useable chassis adapted to receive an absorbent insert therein. The chassis comprises insert fastener components and the absorbent insert comprises a pair of elasticized standing cuffs.

Co-pending application PCT/EP2023/057846 (Pelz, unpublished) describes a system with a re-usable and washable article chassis that is adapted to receive an absorbent core, so as to ease replacing of the core upon loading, addressing improved positioning but also exchange of a loaded absorbent core.

EP0549988 (K-C) describes a disposable article, such as a diaper, for absorbing and containing urine and other body exudates having an absorbent insert pad that may be flushed in a toilet and which may be positioned within a pocket. A cover is releasably attached to a bodyside liner to maintain the insert pad within the pocket. The insert pad includes an absorbent material, which may include wood pulp fluff and high-absorbency materials, such as well-known superabsorbent materials. The absorbent material may be wrapped in a flushable, dispersible carrier sheet, as may comprise a low-wet-strength cellulosic tissue or a nonwoven material such as a rayon carded web that is bonded with polyvinyl alcohol. The ends of the carrier sheet may be folded onto themselves and bonded using sonic bonds, adhesives, such as time sensitive binder such as a polyvinyl alcohol-based adhesive. The longitudinally-extending sides of the carrier sheet may overlap slightly and are preferably unbonded to allow absorbent material to escape from within the carrier sheet once the insert pad has been deposited in a toilet.

However, in course of recent developments, further criteria have been developed to address flushability of nonwoven materials, see EDANA flushability requirements for materials and wipes, EDANA GD.4. For a product to be deemed flushable there must be evidence indicating that
it quickly disintegrates and does not clog toilets and properly maintained drainage pipe systems, passes through wastewater conveyance systems and is compatible with wastewater treatment, reuse and disposal systems without causing system blockage, clogging or other operational problems; and
is unrecognizable in effluent leaving on-site and municipal wastewater treatment systems and in digested sludge from wastewater treatment plants that are applied to soil, essentially satisfying certain bio-degradability criteria.

However, when adopting the flushability criteria to hygiene articles as a combination of such materials all satisfying these criteria, a first challenge remains of not introducing structural elements that induce non-disintegration of the article. Such elements can be any water-insensitive connecting, but also the above-described water-soluble glues may lose adhesive properties in excess of water too slowly.

A further challenge for designing flushable absorbent products relates to longer wearing period of hygiene articles compared to the wipe products for which the criteria have been developed, in particular as during the prolonged wear period wetting of the article results in change of properties, especially reduction of mechanical strength respectively integrity of the article or its elements during use.

Thus, the present invention gives a solution by providing a hygiene article system comprising a quickly disintegrating flushable absorbent pad as may be combined with a particular hygiene chassis comprising a pocket adapted to receive such a pad.

### Summary

In a first aspect, the present invention is an absorbent pad, exhibiting a length direction exceeding its width direction and a thickness perpendicular thereto, comprising an absorbent core comprising fibers and absorbent particles, which exhibits along the length direction of the absorbent pad a dry stiffness of less than 20°, preferably less than 15°, more preferably less than about 10°, according to the Cantilever test, as described herein, and a wet stiffness at 50 % of its design capacity, as defined herein of less than about 12 cm, preferably less than about 10 cm, more preferably less than about 9 cm, each to reach a bending angle of 41.5° according to the Cantilever test, as described herein. The absorbent pad further comprises a wrap web and a removal aid.

Essentially all of the materials of the absorbent pad are satisfying EDANA GD.4 flushability test requirements, adapted to the materials as described herein. Further, a wrapping of the absorbent core by the wrap web, is executed such that at least one surface of the absorbent core is at least partially covered by at least two layers of the wrap web.

Preferably, the absorbent pad is satisfying one or more of the requirements selected from the group consisting of
a) the absorbent pad being essentially free of adhesive or thermoplastic bonding;
b) the absorbent pad being essentially unbonded or by mechanically induced hydrogen bonding only;
c) the absorbent pad exhibiting an absorbent centrifuge capacity of
   c1) more than about 500 ml/m², preferably more than about 1000 ml/m², more preferably more than about 2000 ml/m², and most preferably of more than about 3000 ml/m²; or
   c2) less than 30000 ml/m². preferably less than about 20000 ml/m²;
d) the absorbent pad comprises absorbent particles based on modified starch;
e) the absorbent pad comprises cellulose based fibers;
f) the absorbent core comprises wetness sensitive embossing pattern;
g) the wrapping is essentially unbonded or by mechanically bonding induced hydrogen bonding and/or hydroentangling only;
h) the wrapping being essentially free of adhesive or thermoplastic bonding;
i) the wrapping comprises overfold lines of the wrap web that are completely circumscribing the absorbent core;
j) the wrap web comprises a wetness sensitive embossing pattern.

The removal aid of the absorbent pad preferably comprises more than two layers of the wrap web, and may further comprise one or more of the elements selected from the group consisting of embossing, folding, and comprising a water dispersible or soluble glue.

An absorbent pad according to the present invention may further satisfy one or more of the following requirements selected from the group consisting of
k) flushability criteria as described herein;
l) industrial composting according to DIN EN 13432;
m) garden composting according to NF T51-800:2015.;
n disintegrating faster than the wrap web in the slosh box test included in the GD.4 requirements.

In another aspect the present invention is a hygiene article system comprising an absorbent pad and a re-usable chassis comprising a pocket adapted to receiving the pad, wherein the absorbent pad does not disintegrate upon submitting the system with an absorbent pad inserted into the pocket to the slosh box test as modified to apply to an article.

### Brief description of the Figures

Fig. 1A to D depict principles of a two-piece system with an absorbent pad to be positioned in a chassis.
Fig. 2A to D depict executions of an absorbent pad suitable for the present invention.
Fig. 3 depicts a particular execution according to the present invention.
Fig. 4A to D depicts another particular execution according to the present invention.
Fig. 5A and B shows a preferred feature of a removal aid according to the present invention.
The figures are schematically only, and not to scale. Same numerals refer to same or equivalent features or elements, single (`) or multiple (", "', ...) apostrophes indicate duplicate features, such a left and right or front and back, etc.

### Detailed description

In a first aspect, the present invention is an absorbent pad, which is suitable for being flushed in a toilet and exhibits
a moderate absorbency;
immediate disintegration;
complete biodegradability;
and good manageability for a user, both in a dry and in a wet state.

Thus, an absorbent pad should satisfy the flushability criteria as established for flushable wipes, as summarized in the EDANA specification GD.4 or equivalent, adapted for be useful for complete articles. Optionally, such an absorbent pad should also be suitable for being disposed via the composting route, be this by satisfying the requirements for industrial composting according to DIN EN 13432 or equivalent, or even for home or garden composting, according to NF T51-800:2015, or equivalent.

However, functional liquid absorbency during use and rapid disintegration are design contradictions. Thus, the present invention is an absorbent pad, which in itself is not suitable for use, but regains usability when being combined with a chassis that ensures functional integrity of the absorbent pad in the absorbent chassis, however, when the absorbent pad is removed by the user from the chassis, it readily disintegrates and disperses in an excess of water, such as in a toilet bowl.

The use of such a flushable pad is typically restricted to situations without heavy liquid loadings, as may be overnight situations, but rather suit persons - be these male or female - with low to moderate liquid loss of urine and/or menses, who are sufficiently mobile to use toilets, where they may relieve themselves of larger volumes and also can replace a loaded pad.

Thus, in this first aspect, the present invention is a flushable absorbent pad suitable for the second aspect of the present invention, namely a combination of a flushable absorbent pad with an article chassis.

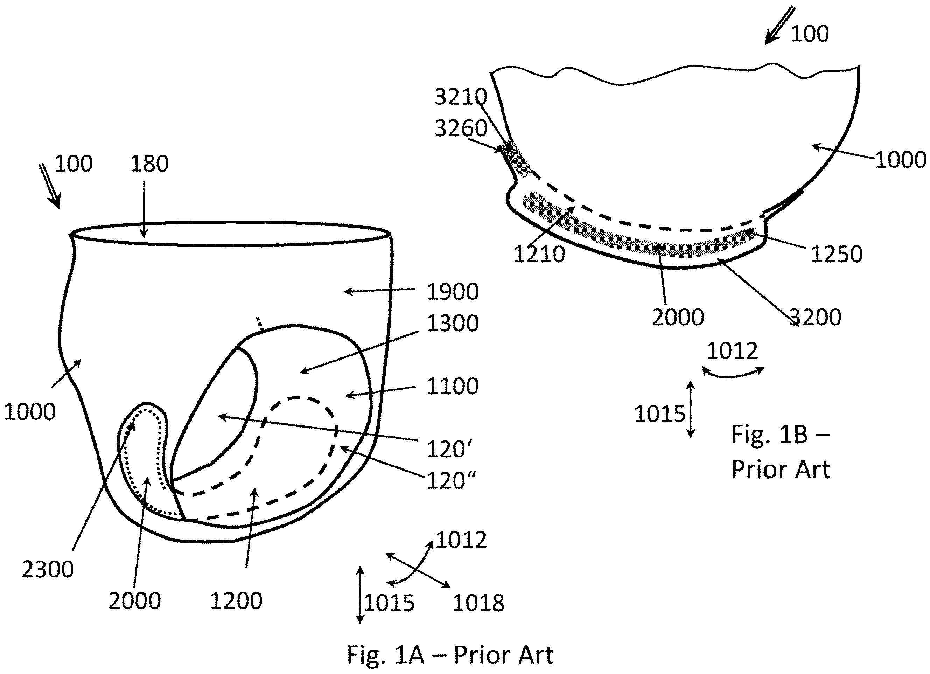

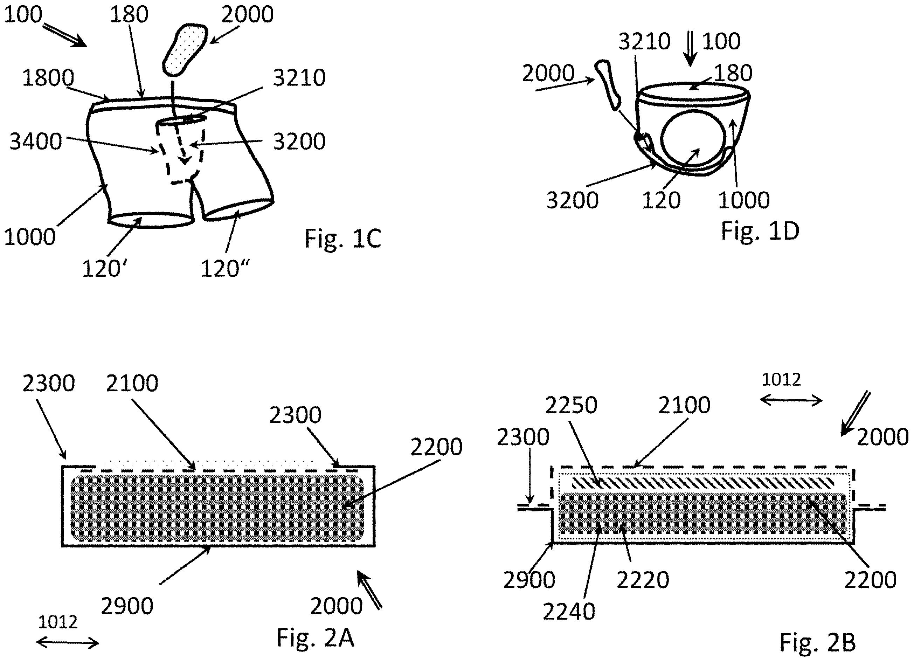

The principles of the present invention are now explained when referring to Fig 1A to D, and 2A to 2D, which however, should not be seen limiting to the invention.

As depicted - and generally known in the art - and absorbent pad 2000 can be positioned into a chassis 1000, and the combined system 100 with a waist opening 180 and two leg openings 120 can be worn by the user. In a preferred execution, and as will be explained in more details herein below the chassis 1000 comprises a pocket 3200 with a pocket opening 3210, optionally with a pocket opening closure means 3260, into which the absorbent pad 2000 can be inserted.

When the absorbent pad is loaded, it can be removed by the user, and a fresh pad can be inserted. The article chassis preferably comprises a main region 1300 and an intake zone 1200, the latter to be generally positioned in registry with the pocket 3200 and the absorbent pad 2000 to be positioned therein.

The article chassis 1000 exhibits a width (y-) 1018 and a length (x-) 1012 direction, the first corresponding to a left-right orientation on a user, the second corresponding to a line extending form the front, i.e., navel, through the crotch to the back, i.e., small of the back, of a user. A thickness (z-) direction 1015 extends perpendicularly to both, whereby the terms "upper" / "inner" and "lower" / "outer" correspond to "towards the user" and "away from the user", respectively. The skilled person will readily apply such terms also to a chassis and an absorbent pad when detached from a user, such as when manufactured or packaged.

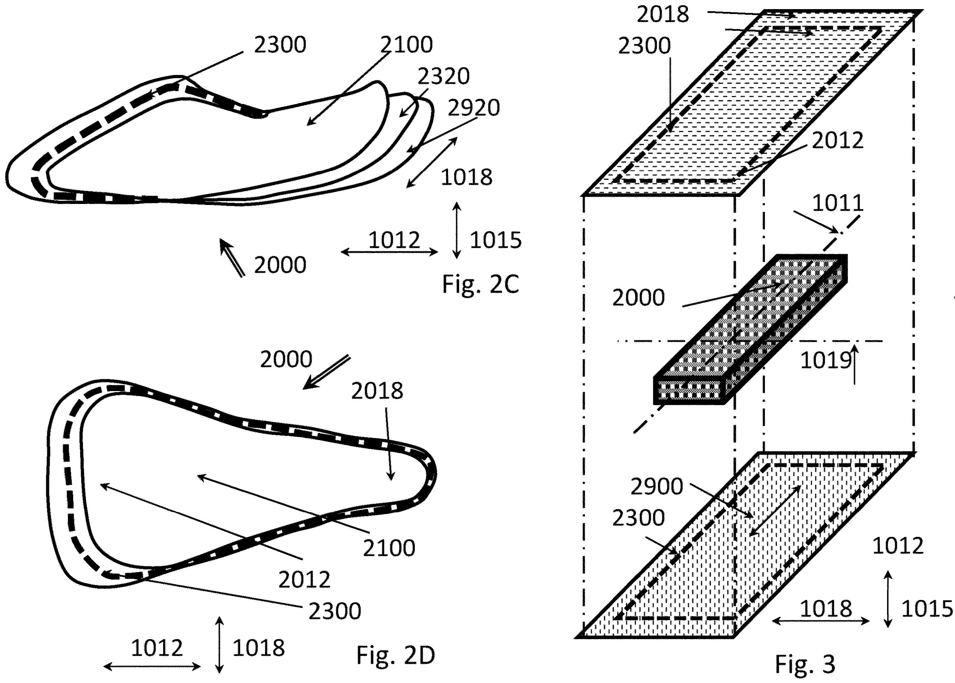

Generally, an absorbent pad and the chassis into which it may be inserted, as shown in Fig. 2A and B in a cross-sectional view along the cross-directional 1018 center-line 1019 as shown further in Fig. 2C with a partly exploded view and in Fig. 2D in a top view, comprises an absorbent core 2200 and optionally a fluid handling aid 2250, sometimes also referred to as an acquisition-distribution layer between a user-oriented cover 2100, often referred to as topsheet, and an opposite cover 2920. Whilst the shape of the absorbent pad is not particularly limited, it is preferred to be elongate, with the longer extension corresponding to the length direction 1012, thus exhibiting a front part 2012 and a rear part 2018 along the orientation as to be worn on a user, i.e., the front part being forwardly oriented away from the crotch portion of the pad. Another particular shape is depicted in Fig. 2C and 2D as a so called "saddle shape".

Similarly, an absorbent pad 2000 according to the present invention comprises a liquid absorbent core 2200, which is wrapped or enveloped in a wrap material, as may be made up by the topsheet 2100 and the backsheet 2900, or be a unitary wrap web 2500, exhibiting an elongated shape along a longitudinal center line 1011, as indicated in Fig. 3.

The absorbent core 2000 is made of materials satisfying the flushability criteria according to EDANA GD.4, optionally also the compostability criteria, as described in the TEST SECTION herein below.

Thus, only fully degradable preferably bio-degradable materials are to be used, such as natural based fibrous materials, such as cellulose based pulp, or viscose rayon, or less preferably synthetic materials that are modified to impart sufficient biodegradability, e.g., as described in WO2022/073764 (Gottlieb Binder), referring to materials such as polycaprolactone, polybutyrate or polybutylene succinate, for which the degradation can be accelerated through the use of enzymes. Further, in order to enhance absorbency properties, particulate material can be added that can absorb water or aqueous solutions at a multiple of its own weight. Such particles well known as "superabsorbent polymers" are currently produced and used at a large scale on a cross-linked polyacrylate basis, however these do not satisfy the degradability criteria.

Highly absorbent powders that do satisfy the (bio-)degradability criteria are known in the art, are often based on mildly crosslinked starch, such as from GreenThings GmbH, Germany - FAIRGREEN biologischer Superabsorber, or Magic srl. Novara, Italy under the trade designation Spongel.

Even though the absorbency and liquid handling properties are often somewhat inferior to the ones of the polyacrylate based ones, these may very well be suited for the present invention, as the absorbent pad can typically be changed more often than a high liquid load pad, such as for overnight loading, would require.

An absorbent core useful for the present invention exhibits an area specific absorbent centrifuge capacity of more than about 500 ml/m² or more than about 1000 ml/m², or more than about 2000 ml/m², even more than about 4000 ml/m², and for certain applications more than about 10000 ml/m², but typically less than about 30000 ml/m².

Highly absorbent powders and fibers can be readily combined into an absorbent composite whereby the particles are intermixed with natural fibers, which is then compressed, such as with a dot pattern, which leads due to the low amounts of residual moisture of the fibers to hydrogen bonds between the fibers of the composite, thereby also entrapping the particles.

Upon wetting, the hydrogen bonds give, and the particles and fibers are released as a first step towards dispersion and subsequent flushability and degradability.

An exemplary absorbent core may be an embossed airlaid material of cellulose fibers with degradable particulate highly absorbent material, whereby the embossing imparts hydrogen bonding between the fibers and thus provides dry integrity but dispersibility upon wetting. Hydrogen bonded air laid materials (HBAL) are generally available such as from McAirlaid's, Germany, under the designation SuperCore SCP-150-TCF, and are also used for the manufacturing of flushable wipes.

An absorbent core preferably exhibits a balanced stiffness. Preferably, the stiffness is such that the application of the absorbent pad with the absorbent core is eased, i.e., exhibiting a dry stiffness according to the Cantilever stiffness test, as described in the TEST SECTION of less than about 20°, or less than about 15°, or less than about 10°, or even less than about 5°.

During and after use, the stiffness should be low to ease removal. Thus, at a load of 50% of 0.9% saline solution of its design capacity, as may be determined by centrifuge capacity, the absorbent pad should exhibit an overhang length to reach a bending angle of 41.5° of less than about 12 cm, or less than about 10 cm r less than about 9 cm, or less than about 8.5 cm.

However, any such an absorbent core that
may lose particulate material even in a dry state,
may start to disintegrate during use upon liquid load,
and must completely disintegrates in an excess of water,
it is important that it is enveloped in a wrap material.

Thus, an absorbent pad according to the present invention comprises a wrap material that may be made of a liquid permeable user-oriented cover or topsheet and an opposite cover or backsheet that may be liquid permeable or impermeable, depending on whether the pocket region of the chassis provides a liquid barrier for protecting wearer's clothing or not. In case of a liquid impermeable opposite cover, care need to be taken that the disintegration - or dissolving - of the liquid barrier material is sufficiently fast to also satisfy the disintegration test.

Other suitable materials may be selected from the class of hydroentangled materials, wherein the fibers are entwisted so as to create integrity without additional adhesives. However, the degree of entanglement and the selection of fibers should be selected so as to satisfy the disintegration criteria. Alternatively, the temporary bonding of the wrap material may also be achieved by selecting materials that allow for hydrogen bonding through compression or crimping, as described for the core, such as well-known from tissues, such as toilet tissues.

Such materials may be delivered as roll stock goods, such as from Suominen, Finland, under the designation Hydraspun ^{®} Aquaflow, or Glatfelter, Germany, under the designation DYNAWEB HE G1.5, as made according to the teaching of WO2016/023856, at a basis weight of 55 g/m² or 60 g/m², with target strength values as shown in Table 1.

Such materials are also readily dispersible, by reaching the dispersibility requirement according to the "slosh box test" as per EDANA GD.4 already at less than 30 mins as compared to the minimum requirement of 60 mins.

Another suitable material from EP'988: One such material, as was sold under the trade designation PRK 20 from Bonded Fiber Fabrics, now Berry Plastics, exhibited approximate tensile strength values also included in Table 1.

**Table 1**

| | Dynaweb BW HE G1.5 | Boded Fiber Fabrics |
|---|---|---|
| Tensile strength MD dry | More than 450 [N/m] | 2950 |
| Tensile strength CD dry | More than 200 [N/m] | 320 |
| Tensile strength MD wet | More than 100 [N/m] | 140 |
| Tensile strength CD wet | More than 65 [N/m] | 7 |

It is an important aspect of the absorbent pad according to the present invention that the core is enveloped by a core wrap construction that is water sensitive and readily loses the wrapping function when exposed to an excess of water.

When separate materials for the topsheet and backsheet are to be used as shown in Fig. 2 and 3, these may be connected to each other, such as along the pad periphery 2300. However, the connecting needs to be water sensitive at least in excess of water so as to not hinder the disintegration of the pad after use, such as by employing water sensitive glues as well known in the art, such as available from Intercol BV, The Netherlands, or Beardow & Adams Ltd, UK, or BC Adhesives, WI, USA.

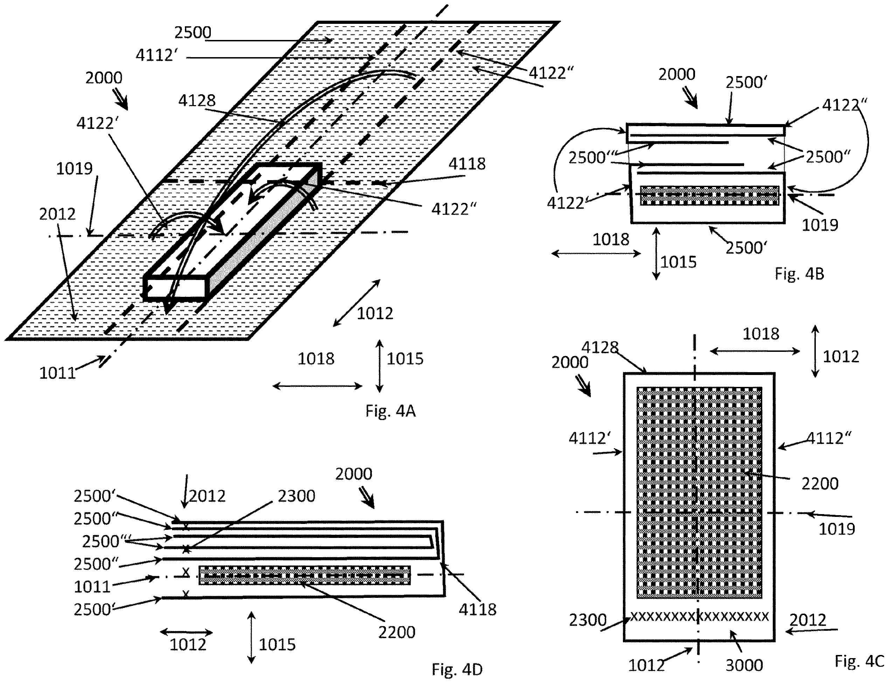

Whilst carefully selected water sensitive may be very suitable for the present invention, these do carry a disadvantage of processing complication, as drying may be required, but also of inducing a higher digestion burden in any water treatment unit after being flushed though the toilet system. Thus, in a particular preferred execution, see Fig. 4 A to D for explanatory, but not limiting purposes, the absorbent pad 2000 according to the present invention comprises a single core wrap web 2500, which functions as user-oriented topsheet as well as opposite backsheet cover of the core 2200 simultaneously and which is folded around the core 2200 by overfolding lines, wherein overfolding refers to a 180° fold, such that the inner surface of a core wrap web material is facing itself after the fold, spaced apart by the absorbent core if positioned therebetween. In the exemplary execution, the core wrap is folded around two longitudinal fold lines 4112' and 4112" and around a cross-directionally extending further fold line 4118. Fig 4A depicts a perspective view prior to the folding along folding arrows 4122', 4122" and 4128, whilst Fig. 4B and D depict slice plane views and Fig. 4C a top view. As depicted, the core wrap protrudes at the front end 2012, where it may be bonded by water sensitive bond 2300, e.g., by crimp induced hydrogen bonding.

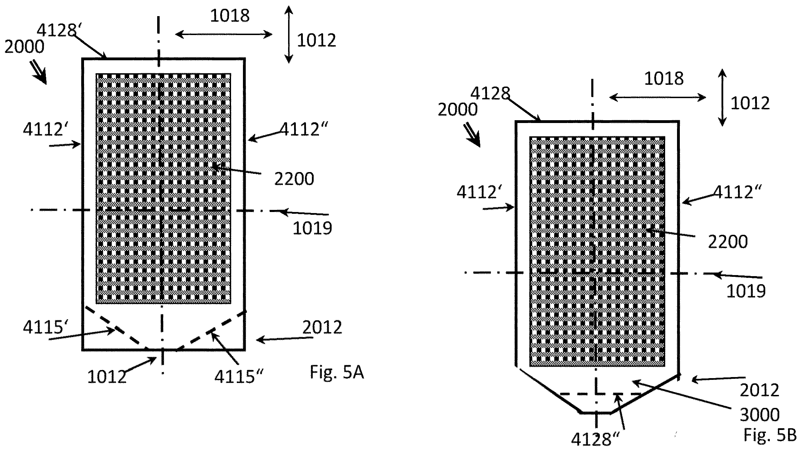

Fig. 5A and B depict top views of an alternative option, wherein the bonding 2300 is replaced by a further trapeziform folded section, wherein the frond side corners are folded along two diagonal fold lines 4115' and 4115", and the resulting triangle tip 2013 is further overfolded along a cross-directional fold line. Such a design provides an option that does not include any core wrap bonding, but the core integrity and containment is achieved by "completely circumscribing by folding lines" refers to a construction wherein any path starting in the core towards the environment, as can be imagined as the one of a migrating particle, is deflected at a fold line, as a barrier for such an imaginary particle. Such a construction is well known from e.g., gift wrapping or from tea bags, however typically further comprising connection means, such as sticky tapes, or staples, or stitching.

Preferably, the wrapped absorbent pad exhibits a tensile strength along its length direction that is more than twice the tensile strength of the wrap web, measured along the direction corresponding to the length direction of the absorbent pad, preferably more than about 2.5 times, or more than about 3 times, or even more than about 6 times.

The wrapped absorbent pad preferably exhibits a dry stiffness corresponding to the dry stiffness of the absorbent pad.

In a loaded state during use, e.g., at a 50% load relative to the centrifuge capacity of the absorbent pad, the stiffness of the core is significantly reduced due to the wetness sensitivity of the core materials.

It is important that the absorbent pad according to the present invention can be readily removed and disposed, such as into the toilet bowl and hence it comprises a removal aid in its front part of the absorbent pad.

Such a removal aid may be a separate material - also satisfying the flushability criteria - as may be connected to the wrap web by the above-mentioned crimping method, such as a cotton thread. Preferably, the removal aid is an extension of the wrap web into the front region. As shown in Fig. 4C, the removal aid 3000 may be formed by the overlapping core wrap in the front region, where the core wrap layers are connected to each other by water sensitive connecting means 2300. Additionally, or alternatively, as shown in Fig. 5B, the removal aid 3000 may be formed by the tea-bag-like overfolding in the front region 2012.

In the further aspect of the present invention, the absorbent core as being fitted into an article chassis is given support to not disintegrate in spite of the loading and wetting during use.

Such a pad is complemented by a chassis that compensates for some in-use deficiencies of a flushable pad.

To this end, the chassis comprises a pocket adapted to receive an absorbent pad in its dry state, where it preferably exhibits a stiffness or rigidity to allow easy insertion. The pocket of such a chassis containing an absorbent pad according to the present invention will provide sufficient protection against mechanical stresses as induced under normal wearing conditions, and hence ensures that the absorbent pad will not lose liquid handling performance due to strong deformation and especially due to premature disintegration of the absorbent core. In a particular execution, the pocket can expand so as to allow for swelling of the absorbent pad during use. Such an expansion can be achieved by using elastic or at least extensible material, wherein the first refer to materials that comprise elastomeric materials and thus exhibit higher retractive forces than the latter, as may be made from essentially in-elastic materials, such as cotton yarns, that are manufactured into a web, such as by knitting, the is flexible and extensible with moderate retraction forces, such as known from e.g., cotton underwear.

Further, the chassis of a system according to the present invention may provide liquid sealing functionality, especially if the absorbent pad does not comprise a built-in liquid barrier layer on the its opposite side. Preferably, the chassis may comprise further liquid barrier properties, such as wicking barriers for preventing lateral liquid distribution.

The stiffness of the chassis is less relevant but should satisfy normal wearing comfort requirements and not hinder insertion of the absorbent pad. A further highly preferred feature of the chassis is that it should not overly occlude the skin, but rather allow aeration and even wetness removal such as by being breathable, preferably by comprising material exhibiting a moisture vapour transmission rate of more than about 2000, or ore than about 7500, or more than about 10000, or even more than about 12000, all expressed in g/m²/24hrs.

Preferably, the chassis comprises breathable fabric or textile webs. Such chassis - pad systems are generally well known in the art, see above referenced US8192415(P&G) for disposable chassis, or more preferably for a washable and reusable chassis, as described in more detail in copending application PCT/EP2023/057846 (Pelz), to which express reference is made for the design and construction of a chassis for receiving an absorbent pad.

### Test methods

### EDANA GD.4

EDANA, Belgium, has established flushability criteria especially for nonwoven materials like wipes, comprising the criteria of
FG501.R1(18): Toilet and Drain-line Clearance Test
FG502.R1(18): Slosh Box Disintegration Test
FG503.R1(18): Household Pump Test
FG504.R1(18): Settling Test
FG505.R1(18): Aerobic (A) Biodisintegration/(B) Biodegradation Tests
FG506.R1(18): Anaerobic (A) Biodisintegration/(B) Biodegradation Tests
FG507.R1(18): Municipal Sewage Pump Test

The skilled person will readily realize that articles - such as absorbent pads - should satisfy the same criteria in tests just adapted to cope with potentially different shape of the article. Similarly, also the tests may be applied to materials as used for the construction of such articles, also adapted to reflect the different shape - for example, particulate or loose fibrous material will automatically satisfy the Slosh box Disintegration Test.

When applying the "Slosh Box Disintegration Test" to a system comprising an absorbent pad and a chassis, the absorbent pad should be inserted into the pocket of the chassis without unduly deforming the absorbent pad.

Industrial composting according to the requirements of DIN EN 13432.

Home or garden composting according to NF T51-800:2015

Tensile strength according to EDANA NWSP 110.4.R0 (15), at 25 mm strip width and constant rate of extension.

Cantilever Stiffness Test EDANA NWSP 090.1 (R0 (15)

At the dry state, the angle for an overhang length of 160 mm is recorded.

For the wet state, the material or article was loaded with 0.9% saline solution up to 50% of its design capacity, as may be determined by the centrifuge capacity absorption, and the overhanging length to reach an angle of 41.5° is recorded

Design capacity by Centrifuge capacity EDANA NWSP 241.0 R0 (15), as may be adapted such that the particulate material is replaced by a web material.

Moisture vapour Transmission Rate according to ASTM E96-80, expressed in g/m²/24hrs.

## Claims

1. An absorbent pad,
exhibiting a length direction exceeding its width direction and a thickness perpendicular thereto, comprising
an absorbent core
comprising fibers and absorbent particles,
exhibiting along the length direction of the absorbent pad
a dry stiffness of less than 20°, preferably less than 15°, more preferably less than about 10°, according to the Cantilever test, as described herein, and
and a wet stiffness at 50 % of its design capacity, as defined herein of less than about 12 cm, preferably less than about 10 cm, more preferably less than about 9 cm, each to reach a bending angle of 41.5° according to the Cantilever test, as described herein;
a wrap web;
a removal aid;
consisting essentially of materials satisfying EDANA GD.4 flushability test requirements, adapted to the materials as described herein,
further comprising a wrapping of said absorbent core by said wrap web,
such that at least one surface of said absorbent core is at least partially covered by at least two layers of said wrap web.

2. An absorbent pad according to claim 1, satisfying one or more of the requirements selected from the group consisting of
a) said absorbent pad being essentially free of adhesive or thermoplastic bonding;
b) said absorbent pad being essentially unbonded or by mechanically induced hydrogen bonding only;
c) said absorbent pad exhibiting an absorbent centrifuge capacity of
c1) more than about 500 ml/m², preferably more than about 1000 ml/m², more preferably more than about 2000 ml/m², and most preferably of more than about 3000 ml/m²; or
c2) less than 30000 ml/m². preferably less than about 20000 ml/m²;
d) said absorbent pad comprises absorbent particles based on modified starch;
e) said absorbent pad comprises cellulose based fibers;
f) said absorbent core comprises wetness sensitive embossing pattern.

3. An absorbent pad according to any of the preceding claims, further comprising one or more of the elements selected from the group consisting of
g) said wrapping is essentially unbonded or by mechanically bonding induced hydrogen bonding and/or hydroentangling only;
h) said wrapping being essentially free of adhesive or thermoplastic bonding;
i) said wrapping comprises overfold lines of said wrap web that are completely circumscribing said absorbent core;
j) said wrap web comprises a wetness sensitive embossing pattern.

4. An absorbent pad according to any of the preceding claims, further comprising a removal aid, preferably comprising more than two layers of said wrap web, comprising one or more of the elements selected from the group consisting of embossing, folding, and comprising a water dispersible or soluble glue.

5. An absorbent pad according to any of the preceding claims, further satisfying one or more of the following requirements selected from the group consisting of
k) flushability criteria as described herein;
l) industrial composting according to DIN EN 13432;
m) garden composting according to NF T51-800:2015.;
n disintegrating faster than said wrap web in the slosh box test included in said GD.4 requirements.

6. A hygiene article system comprising
- an absorbent pad according to any of the preceding claims
and
- a re-usable chassis comprising a pocket adapted to receiving said pad,
wherein said absorbent pad does not disintegrate upon submitting the system with an absorbent pad inserted into said pocket to the slosh box test as modified to apply to an article.
